# EUROPEAN PATENT SPECIFICATION

(11) **EP 1 439 831 B1**
(45) Date of publication and mention of the grant of the patent: **15.06.2005**
(21) Application number: 02772745.2
(22) Date of filing: 15.10.2002
(51) Int. Cl.: A61K 31/197, A61K 31/198, A61K 31/7052, A61K 38/00, A61K 31/195, A61P 3/02, A61P 39/06

(54) **PHARMACO-DIETARY PREPARATION HAVING A NUTRITION-SUPPLEMENTING AND NUTRITION-ENHANCING EFFECT**
PHARMAKOLOGISCHE NAHRUNGSZUSAMMENSETZUNG ZUR NAHRUNGSERGÄNGZUNG UND NÄHRWERTVERBESSERUNG
PREPARATION NUTRACEUTIQUE AYANT UN EFFET DE NUTRITION D'APPOINT ET D'AMELIORATION DE NUTRITION

(30) Priority: 01.11.2001 GB 0126194
(43) Date of publication of application: 28.07.2004
(73) Proprietor: New Technology Research Ltd., Tortola (VG)
(72) Inventor: RAGGI, Giuseppe, CH-6900 Lugano (CH)
(74) Representative: Fiammenghi-Domenighetti, Delfina
(86) International application number: PCT/IB2002/004242
(87) International publication number: WO 2003/037320

(56) References cited:
- EP-A- 0 891 719
- WO-A-95/31114
- WO-A-99/65476
- FR-A- 2 154 397
- US-A- 5 569 458
- US-A- 5 587 399
- US-A- 5 626 883
- US-A- 5 656 608
- US-A- 5 925 377

## Description

The present invention relates to pharmaceutical and/or dietary compositions and/or functional human and/or animal foods capable of promoting a reduction of excess weight, preventing aging processes, and assisting in the treatment of disorders linked thereto: atherosclerosis, hypertension, diabetes, osteoporosis, menopausal syndromes, senile cerebral disorders (Alzheimer's disease, Parkinson's disease, dementias and memory losses), psychophysical stresses, depression, chronic fatigue syndrome, cutaneous and dermal aging (wrinkles, cellulitis, alopecia, et cetera), benign prostate hypertrophy, et cetera.

Free radicals (and the peroxidative processes they induce), together with protein malnutrition (often caused by inefficient digestion of proteins and/or by a reduced efficiency of intestinal absorption of amino acids) and with deficits of vitamins, oligoelements and minerals and vitamin-like factors (for example nucleosides derived from the digestion of nucleic acids), have long been recognized as the primary causes of metabolic and structural alterations (such as excess weight, high plasma levels of cholesterol, triglycerides, glucose, reduced levels of antioxidant defences in plasma and in the various tissues, energy deficits of mitochondria and of cell metabolism, damage to DNA and RNAs) that occur in various situations of psychophysical stress and during aging, as well as during the onset of many disorders correlated to aging such as atherosclerosis, diabetes, hypertension, et cetera (Supplement to "The American Journal of Clinical Nutrition", vol. 53 (No. 1), 1991, p. 189; "Lipid Peroxidation": part II: "Pathological Implications", 1987, Chemistry and Physics of Lipids, vol. 45 (no. 2-4), p. 103; "Undernutrition in elderly people", 1989, Age Ageing, vol. 18, p. 339; "Malnutrition and falls", 1990, Lancet, vol. 336, p. 1447).

In order to avoid all these pathological degenerations, the inventor of the present invention has devised a preparation that has marked organoleptic virtues. The invention is in fact constituted by a preparation as described in the accompanying Claim 1.

A description is now given of some preferred embodiments of the preparation according to the invention.

The composition of the preparation according to the invention essentially comprises:
- a hydrolysate of amino acids and/or peptides, with a relative molecular mass between 10² and 2 x 10⁴ daltons, obtained by hydrolysis of proteins having a high biological value (for example proteins of milk serum, soybean, eggs, wheat, maize, yeasts, fish, meat, et cetera) with the addition of β-alanine in an amount ≥ 0.1% of the aminoacyl total and preferably between 1 and 3%. Said hydrolysate must receive a further addition of glycine (≥ 1.5% of the aminoacyl total) and/or glutamine (≥ 3% of the aminoacyl total and/or taurine (≥ 0.1% of the amino acyl total) and/or arginine (≥ 2.1% of the aminoacyl total) if these amino acids are not already present in the above cited amounts.

To boost the effects of the preparation according to the invention it is possible to add:
- a hydrolysate of oligonucleotides and/or nucleotides and/or nucleosides, obtained by hydrolysis from ribonucleic and/or deoxyribonucleic acids extracted from yeasts, plants, meat or fish, with a relative molecular mass preferably between 10² and 10⁴ daltons, optionally with the addition of adenosine (so that the amount of adenine is ≥ 10% of the total of all the nitrogen bases present in the oligonucleotides and/or nucleotides and/or nucleosides of the hydrolysate).
- a mixture of protein extracts having a hydrolytic activity, of plant and/or animal and/or bacterial origin (for example extracts of *Aspergillus oryzae* fermented in the presence of rice starch).
- a mixture containing D-ribose and/or xylitol.

Furthermore, the preparation according to the invention can contain other components used conventionally, such as for example:
- the different species of vitamins and/or vitamin-like products (for example carnitine, creatine, carnosine, homo-carnosine, anserine, betaine, lipoic acid, essential fatty acids of the w-6 and w-3 series, lecithins, inositol, et cetera)
- the various species of minerals and oligoelements
- carbohydrates of various kinds (glucose, fructose, saccharose, lactose, arabinose, starches, maltodextrins, et cetera)
- indigestible fibres and/or polysaccharides (inulins, pectins, celluloses, cyclodextrins, et cetera)
- extracts of plants and/or spices and/or medicinal plants containing phytosterols, bioflavones, terpenes, essential oils, et cetera.

As regards the dosage of the various compounds of the preparation, it can be defined within a wide discretionary range: however, the inventor suggests, for the preparation, a dosage of 0.05 ö 5.0 grams of preparation per day per kilogram of body weight of the person taking it, although optimum dosage is between 0.5 and 2.0 grams per day per kilogram of body weight.

As regards the relative dosage of the individual components of the preparation, the inventor suggests to use preferably an amount of said hydrolysate of oligonucleotides and/or nucleotides and/or nucleosides between 1 and 10 mg per day per kg of body weight.

For said mixture of protein extracts having hydrolytic activity of plant and/or animal origin, the inventor suggests a dosage between 0.01 and 2 grams, but preferably between 0.1 and 0.5 grams, per kg of body weight per day.

For said mixture containing D-ribose and/or xylitol, moreover, the inventor suggests a daily dosage in which the administered amount of D-ribose is 0.1 to 250 mg, but preferably 1 ö 25 mg, per kg of body weight, and the amount of xylitol is 0.1 to 1000 mg, but preferably 2 to 100 mg, per kg of body weight. Obviously, the preparation according to the invention can be administered as a single daily dose or split into multiple doses.

The powder and/or granulated forms of the above described components, which are perfectly miscible and usable with each other, are formulated in a composition suitable for oral administration, such as sachets containing powders or granulates; pastilles and dragées; ordinary or effervescent tablets; pasta, rice, crackers, bread, biscuits or other bakery products obtainable by mixing the various active ingredients, in the form of powders and/or granulates, with appropriate food-grade pharmacologically insert excipients, such as simple or complex carbohydrates (food-grade flours of various origin, starches, vegetable fibres of various kinds, and celluloses, chitins or chitosans, pectins, inulins, saccharose, lactose, et cetera); sauces, condiments, creams and/or mayonnaises obtainable by mixing the active ingredients with oils, water, lecithin, natural emulsifiers and any other ingredient normally used in this type of preparation; powdered dispersions for extemporaneous production of milk-beverages and yogurth, beverages of various kinds; appropriately flavoured chewing-gums, et cetera.
A preparation according to the invention can also be used in the cosmetic application-field in the form of a cream, of a gel or the like, via aerosol etc.

Two non-limitative examples of possible formulations according to the present invention are presented hereinafter.

### Example 1

A) 100 g of a hydrolysate of amino acids and/or peptides (with a relative molecular mass between 10² and 2x10⁴ daltons) from milk serum proteins, having the following amino acid composition:

| | | | | | |
|---|---|---|---|---|---|
| Alanine | 5.04 g | Leucine | 12.096 g | Tyrosine | 3.444 g |
| Arginine | 2.184 g | Lysine | 9.66 g | Valine | 4.704 g |
| Aspartic acid | 10.164 g | Methionine | 2.101 g | | |
| Cystine | 3.024 g | Phenylalanine | 3.276 g | | |
| Glutamine and glutamic acid | 15.12 g | Proline | 4.368 g | | |
| Glycine | 1.512 g | Serine | 3.024 g | | |
| Histidine | 1.764 g | Threonine | 4.452 g | | |
| Isoleucine | 4.956 g | Tryptophan | 2.100 g | | |

with the addition of
- 4 g glycine
- 6 g glutamine
- 1500 mg β-alanine
- 250 mg taurine
+
B) 2 g of a mixture of oligonucleotides, nucleotides and nucleosides (with a relative molecular mass between 2x10² and 10x10³ daltons) obtained by hydrolysis of nucleic acids from yeast, with the addition of 500 mg of adenosine.
+
C) 8 g of a mixture of protein extracts having a hydrolytic activity (4 g of protein extract of pineapple stalk rich in bromelain + 4 g of pancreatic protein extract rich in trypsin, chymotrypsin, et cetera)
+
D) 5 g of a mixture of D-ribose (1 g) and xylitol (4 g).

### Example 2

A) 127.25 g of a mixture of protein and nucleotide hydrolysates, protein extracts having proteolytic activity and D-ribose and xylitol as in example 1 A) + 1 B) + 1 C) + 1D)
+
B) a mixture of vitamins, vitamin-like factors, minerals and oligonucleotides, carbohydrates and fibres constituted by:

| | | | | | | | |
|---|---|---|---|---|---|---|---|
| Inulins and pectins | 8 g | B6 | 8 mg | Zn | 20 mg | Phosphor | 200 mg |
| Vitamin A | 2 mg | B12 | 200 µg | Cu | 100 µg | Sodium | 300 mg |
| Vitamin E | 100 mg | Biotin | 200 µg | Boron | 100 µg | Maltodextrins | 22 g |
| Vitamin C | 100 mg | Folic acid | 200 µg | Cr | 100 µg | w-6 and w-3 essential fatty acids | 4 g |
| Vitamin D | 12 µg | Inositol | 400 mg | Vanadium | 40 µg | Lecithins | 4 g |
| Vitamin B1 | 4 mg | Ca | 200 mg | Molybdenum | 100 µg | Creatine | 4 g |
| Vitamin B2 | 4 mg | Magnesium | 200 mg | Iodine | 100 µg | Lipoic acid | 200 mg |
| B3 nicotinamide | 60 mg | Potassium | 600 mg | Iron | 4 mg | | |
| B5 pantothenic acid | 20 mg | Chloride | 300 mg | Mn | 10 mg | | |

In order to study the pharmacological and/or dietary characteristics of the composition according to the present invention, a series of experimental tests on rats and clinical tests in man was conducted.

As regards experimental tests on rats, 60 male rats, divided into 5 groups of 12 animals each, were used. Each group of animals was subjected to a dietary regimen as listed in Table I according to times and methods indicated in Table II.

At the end of the treatments, various body composition parameters were measured (initial and final weight, percentage compositions of H₂O, proteins and fats in the body, variation of levels of deposit of epididymal and perirenal fats; Table III; blood levels of total cholesterol, HDL cholesterol, triglycerides and glucose (Table III): levels of lipoperoxides (MDA) in plasma, liver, brain and heart, hepatic content of reduced glutathione (GSH), and consumption of hepatocellular oxygen and renal levels of 8-oxo-d-guanosine (Table IV).

Experimental data listed in Tables III and IV show that:
1) Treatment for 84 days with a diet rich in fats with respect to the standard diet induced a dramatic and significant increase in body weight and fat content of the animal, with a decrease in protein masses and in the state of hydration of tissues. There was also a significant increase in blood levels of triglycerides, cholesterol and glucose. Levels of lipoperoxides in plasma and in the various tested tissues were also increased (a clear indicator of reduced efficiency of antioxidant defences!), and there was also a dramatic decrease in liver content of reduced glutathione (further confirmation of the drop in antioxidant defences!). There was also a reduction in the consumption of hepatocellular oxygen (an indicator of reduced energy efficiency of mitochondrial functions!) and a considerable renal increase in 8-oxo-d-guanosine (an indicator of structural and functional damage to nuclear and/or mitochondrial DNA). All these forms of damage indicated a loss of tissue functionality and predisposition to accelerated aging and to the onset of the dysmetabolic disorders correlated thereto (atherosclerosis, diabetes, hypertension, et cetera).
2) Administration of restricted-calorie diets constituted by milk serum proteins; either untreated (MSP) or hydrolysed (HMSP), was capable of producing a modest preventative effect on the onset of these metabolic-functional alterations.
3) Administration of restricted-calorie diets constituted by hydrolysates of milk serum protein plus the supplements as listed in example 1 of the present invention (HMSP+I) was instead capable of producing a considerable and surprising synergistic effect in:
   - facilitating reduction of excess body weight
   - increasing lean protein mass and decreasing excess accumulated body fat
   - facilitating tissue hydration
   - improving antioxidant defences in plasma and in the various tissues
   - improving the energy-functional efficiency of the mitochondrion
   - reducing damage and mutations affecting nuclear and/or mitochondrial DNA

These therapeutic benefits, obtainable by administering the protein hydrolysates with the addition of the various supplements claimed in the invention, are always significantly greater than the sum of the benefits obtainable by administering separately the protein fractions alone (untreated or hydrolysed, or as the various individual components of the integrated mixture).

In human clinical tests, several groups of overweight individuals, both healthy and affected by one or more of the many dysmetabolic disorders often correlated to aging and/or excess weight (atherosclerosis, diabetes, hypertension, cerebral-degenerative disorders such as Alzheimer's disease, senile dementias, memory loss, et cetera, osteoporosis and menopausal syndromes, states of psychophysical stress, chronic fatigue syndrome, skin aging, wrinkles, cellulite, alopecia, et cetera, benign prostate hypertrophy, et cetera) were subjected to a dietary treatment with the mixture formulated according to the invention as described in example 2. The doses of the mixture and the administration times varied according to the groups being treated and the extent of the initial excess weight.

In all the clinical studies that were conducted, the beneficial and therapeutic effects obtainable by administering the mixtures formulated according to the invention as listed in example 2 were always been found highly significant in reducing excess weight and improving aging predictive indices, in improving antioxidant defences in plasma (evaluated by monitoring the levels of MDA), in reducing damage to nuclear and/or mitochondrial DNA (evaluated by monitoring 8-oxo-d-guanosine in cells of the mucous membrane of the mouth and/or in urine), and in improving the various tested clinical parameters.

These beneficial aspects observable by administering the mixture formulated completely according to the invention were always been far greater than the effects obtainable by administering the various components (individually or in partial association) that constitute the formulated mixture.

The beneficial and therapeutic effects obtained by administering the mixture formulated according to the present invention also proved themselves capable of increasing and synergistically combining the therapeutic benefits obtainable with the drugs normally used in the various tested disorders; hence the evidence of a possible additional benefit of the use of these supplements: their synergistic effects in assisting the therapeutic action of drugs normally in use in the various disorders cited above.

**TABLE I:**

| Percentage composition of experimental diets | | | | | |
|---|---|---|---|---|---|
| **Components** | **Obesity-inducing diet** | | **Restricted-calorie diets** | | |
| | **Standard diet*** | **Fat-rich diet** | **MSP** | **HMSP** | **HMSP + supplements as listed in Example 1** |
| Standard diet | 100.0 | 60.0 | = | = | = |
| Milk serum proteins (MSP) | | | 40 | | |
| Hydrolysed proteins from milk serum (HMSP) | | | | 40 | |
| HMSP + supplements as listed in Example 1 | | | | | 50 |
| Starch | | | 35.3 | 35.3 | 25.3 |
| Saccharose | | | 10 | 10 | 10 |
| Lard + hydrogenated soya oil = (1+3) | | 40 | | | |
| Soya oil | | | 5 | 5 | 5 |
| Cellulose | | | 5 | 5 | 5 |
| Mixture of minerals | | | 3.5 | 3.5 | 3.5 |
| Mixture of vitamins | | | 1.0 | 1.0 | 1.0 |
| Choline bitartrate | | | 0.2 | 0.2 | 0.2 |
| The standard diet is constituted by: casein (20%); starch (55.3%); saccharose (10%); soya oil (5%); cellulose (5%); mixture of minerals (3.5%); mixture of vitamins (1%); choline bitartrate (0.2%). | | | | | |

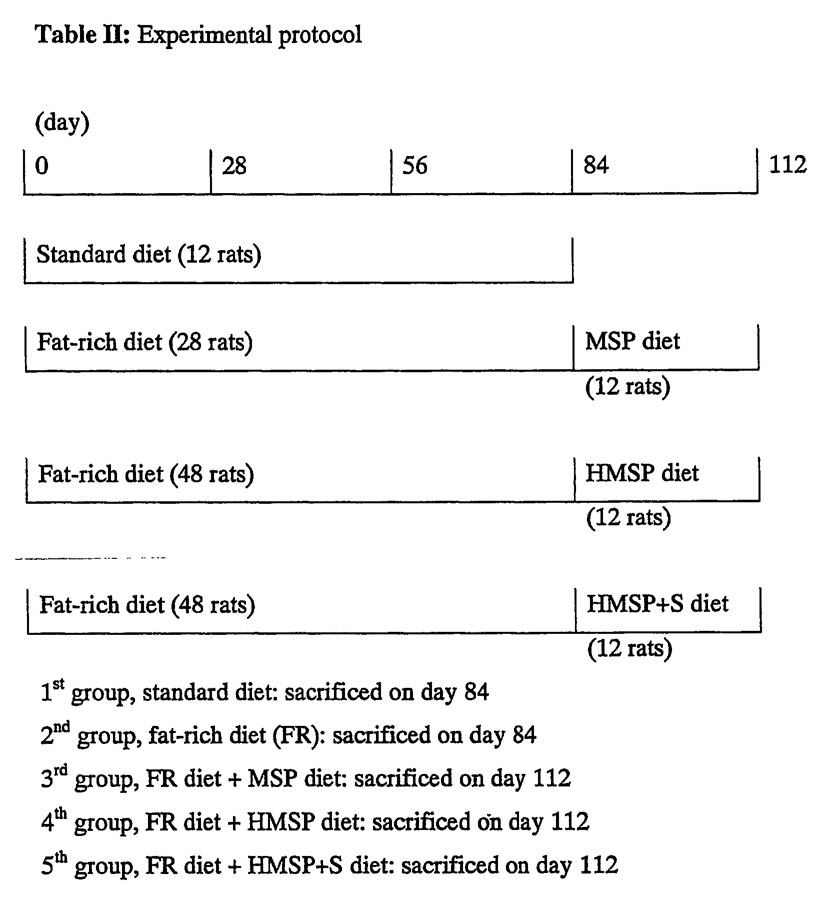

**Table III:**

| Evaluation of the various body composition parameters and blood levels of glucose, cholesterol and triglycerides in rats subjected to the various reference diets and to restricted-calorie diets. | | | | | |
|---|---|---|---|---|---|
| | **Reference diets** | | **Restricted-calorie diets** | | |
| | Standard diet | Fat-rich diet | Serum proteins (MSP) | Hydrolysed milk serum proteins (HMSP) | HMSP + supplements as listed in Example 1 (HMSP + S) |
| Initial body weight (g) | 93.0 | 94.4 | 474.4 | 478.6 | 476.4 |
| Final body weight (g) | 391.5 | 476.2 | 438.4 | 432.4 | 408.5 |
| Daily weight gain or loss (g/day) | +3.5 | +4.5 | -1.2 | -1.6 | -2.4 |
| Epididymal fat content (g/100 g of body weight) | 2.16 | 3.44 | 3.22 | 2.98 | 2.61 |
| Perirenal fat content (g/100 g of body weight) | 2.61 | 4.42 | 3.90 | 3.75 | 3.02 |
| % H₂O content of carcasses | 58.4 | 51.2 | 52.6 | 53.5 | 56.0 |
| % protein content of carcasses | 19.4 | 16.9 | 17.6 | 18.1 | 19.1 |
| % fat content of carcasses | 18.0 | 27.4 | 25.1 | 23.6 | 19.7 |
| Blood glucose (mmol/litre) | 9.87 | 10.54 | 11.26 | 10.68 | 9.21 |
| Blood triglycerides (mmol/litre) | 1.07 | 1.54 | 1.39 | 1.17 | 0.86 |
| Total blood cholesterol (mmol/litre) | 1.68 | 1.94 | 2.24 | 2.12 | 1.78 |
| Blood HDL cholesterol (mmol/litre) | 0.99 | 1.07 | 1.24 | 1.15 | 1.05 |

**Table IV:**

| Levels of lipoperoxides as nmols of malonyldialdehyde (MDA) per g of tissue or per ml of plasma, variations in hepatocellular oxygen consumption and hepatic levels of reduced glutathione (GSH), and variations in kidney levels of 8-oxo-d-guanosine in rats subjected to the various diets. | | | | | |
|---|---|---|---|---|---|
| | **Reference diets** | | **Restricted-calorie diets** | | |
| | Standard diet | Fat-rich diet | Milk serum proteins (MSP) | Hydrolysed milk serum proteins (HMSP) | HMSP + supplements as listed in example I (HMSP+I) |
| *plasma MDA | 2.5 | 5.1 | 5.0 | 4.6 | 3.4 |
| *liver MDA | 25.6 | 44.8 | 41.5 | 36.8 | 28.9 |
| *brain MDA | 55.4 | 108.5 | 102.4 | 98.5 | 76.5 |
| *heart MDA | 24.8 | 45.6 | 40.2 | 36.8 | 29.8 |
| **hepatocellular oxygen consumption µmols O₂/min per 10⁷ cells) | 276.4 | 194.5 | 206.8 | 228.4 | 259.3 |
| **hepatic GSH nmols/10⁵ cells | 36.1 | 22.4 | 28.0 | 32.2 | 40.8 |
| ***Kidney levels of 8-oxo-7,8 dehydro-2'-deoxyguanosine expressed as ratio with respect to d-guanosine (x 10⁵) | 2.87 | 3.65 | 3.56 | 3.48 | 3.08 |

| | | | | | |
|---|---|---|---|---|---|
| * MDA is dosed according to the method of K. Yogi et al., 1982 "Lipid Peroxides in Biology and Medicine", Academic Press, New York, pages 324-340 | | | | | |
| ** O₂ consumption and levels of GSH in liver are dosed according to the method of T. M. Hagen et al., 1999, FASEB J., vol. 13, pages 411-418 | | | | | |
| *** the kidney level of 8-oxo-d-guanosine is dosed according to the method of M. Karbownik et al., 2001, Mutation Res., 474, pages 87-92. | | | | | |

## Claims

1. A pharmaco-dietary preparation having a nutrition-supplementing and nutrition-enhancing effect, comprising:
a hydrolysate of amino acids and/or peptides from milk serum having a relative molecular mass between 10² and 2x10⁴ daltons obtained from proteins, and β-alanine in an amount equal to, or greater than, 0,1% of the aminoacyl total of said hydrolysate of amino acids and/or peptides, **characterized in that** it further comprises:
- a hydrolysate of oligonucleotides and/or nucleotides and/or nucleosides obtained by hydrolysis from ribonucleic and/or deoxyribonucleic acids extracted from yeasts, having a relative molecular mass between 10² and 10⁴, optionally with the addition of adenosine so that the amount of adenine is ≥10% of the total of all nitrogenous bases present in the oligonucloeotides and/or nucleotides and/or nucleosides of the hydrolysate, and/or
- a mixture of protein extracts having a hydrolytic activity of plant and/or animal and/or bacterial origin, and/or
- a mixture containing d-ribose and/or xylitol.

2. The preparation according to claim 1, having the consistency of powder or granulate.

3. The preparation according to claim 1, packaged in the form of tablets.

## Patentansprüche

1. Pharmazeutisch-diätetische Zubereitung mit einer ernährungsergänzenden und ernährungsverstärkenden Wirkung, umfassend:
ein Hydrolysat von Aminosäuren und/oder Peptiden aus Milchserum mit einer relativen Molekularmasse zwischen 10² und 2 x 10⁴ Dalton, das aus Proteinen erhalten ist, und β-Alanin in einer Menge, die gleich oder größer als 0,1 % der Aminoacylgesamtmenge des Hydrolysats von Aminosäuren und/oder Peptiden ist,
**gekennzeichnet dadurch, dass** es ferner
- ein Hydrolysat von Oligonucleotiden und/oder Nucleotiden und/oder Nucleosiden, das durch Hydrolyse von Ribonuclein- und/oder Desoxyribonucleinsäuren, die aus Hefen extrahiert sind, mit einer relativen Molekularmasse zwischen 10² und 10⁴ erhalten ist, gegebenenfalls mit Zusatz von Adenosin, so dass die Adeninmenge ≥ 10 % der Gesamtmenge aller stickstoffhaltigen Basen, die in den Oligonucleotiden und/oder Nucleotiden und/oder Nucleosiden des Hydrolysats vorhanden sind, beträgt, und/oder
- ein Gemisch von Proteinextrakten mit hydrolytischer Aktivität von pflanzlicher und/oder tierischer und/oder bakterieller Herkunft, und/oder
- ein Gemisch, das D-Ribose und/oder Xylitol enthält,
umfasst.

2. Zubereitung gemäß Anspruch 1 mit der Konsistenz eines Pulvers oder eines Granulats.

3. Zubereitung gemäß Anspruch 1, die in Tablettenform abgepackt ist.

## Revendications

1. Préparation pharmaco-diététique, ayant un effet de supplément alimentaire et d'amélioration nutritionnelle, comprenant :
un hydrolysat d'acides aminés et/ou de peptides de sérum de lait, ayant une masse moléculaire relative située entre 10² et 2 x 10⁴ daltons, obtenu à partir de protéines et contenant de la β-alanine en une quantité égale ou supérieure à 0,1% du total aminoacyle dudit hydrolysat d'acides aminés et/ou de peptides, **caractérisée en ce qu'**elle comprend en outre :
- un hydrolysat d'oligonucléotides et/ou de nucléotides et/ou de nucléosides obtenu par hydrolyse d'acides ribonucléique et/ou désoxyribonucléique extraits à partir de levures, ayant une masse moléculaire relative située entre 10² et 10⁴, éventuellement avec addition d'adénosine de sorte que la quantité d'adénine soit ≥ 10% du total de toutes les base azotées présentes dans les oligonucléotides et/ou nucléotides et/ou nucléosides de l'hydrolysat, et/ou
- un mélange d'extraits protéiniques ayant une activité hydrolytique d'origine végétale et/ou animale et/ou bactérienne, et/ou
- un mélange contenant du d-ribose et/ou du xylitol.

2. Préparation selon la revendication 1, ayant la consistance d'une poudre ou d'un granulat.

3. Préparation selon la revendication 1, conditionnée sous la forme de comprimés.
